(19) Europäisches Patentamt
European Patent Office
Office européen des brevets

(11) **EP 2 361 160 B1**

(12) **EUROPÄISCHE PATENTSCHRIFT**

(45) Veröffentlichungstag und Bekanntmachung des Hinweises auf die Patenterteilung:
**26.12.2012 Patentblatt 2012/52**

(21) Anmeldenummer: **09745361.7**

(22) Anmeldetag: **06.05.2009**

(51) Int Cl.:
***B09B 1/00*** *(2006.01)*    ***B09B 3/00*** *(2006.01)*

(86) Internationale Anmeldenummer:
**PCT/CH2009/000143**

(87) Internationale Veröffentlichungsnummer:
**WO 2009/137948 (19.11.2009 Gazette 2009/47)**

(54) **VERFAHREN UND SYSTEM ZUR VERRINGERUNG VON CO2- UND/ODER METHANGAS IN DER ATMOSPHÄRE UND/ODER ZUM ERHALT VON BIOGAS, UND VERWENDUNG DES SYSTEMS**

METHOD AND SYSTEM FOR REDUCING CO2 AND/OR METHANE GAS IN THE ATMOSPHERE AND/OR OBTAINING BIOGAS, AND USE OF SAID SYSTEM

PROCÉDÉ ET DISPOSITIF DE RÉDUCTION DE CO2 ET/OU DE GAZ MÉTHANE DANS L'ATMOSPHÈRE ET/OU D'OBTENTION DE BIOGAZ, ET UTILISATION DU DISPOSITIF

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **14.05.2008 CH 7322008**

(43) Veröffentlichungstag der Anmeldung:
**31.08.2011 Patentblatt 2011/35**

(73) Patentinhaber: **Deco-Hanulik AG**
**8049 Zürich (CH)**

(72) Erfinder: **HANULIK, Jozef**
**CH-8049 Zürich (CH)**

(74) Vertreter: **Felber, Josef**
**Felber & Partner AG**
**Dufourstrasse 116**
**8034 Zürich (CH)**

(56) Entgegenhaltungen:
**EP-A- 0 320 393**          **WO-A-2008/134486**
**DE-A1-102006 045 872**     **US-A- 4 273 615**
**US-A1- 2002 111 527**

EP 2 361 160 B1

**Beschreibung**

Die Kohlendioxidkonzentration in der Luft beträgt heute im Mittel ca. 381 ppm. Aufgrund der global wirkenden Windverfrachtungen sind die geographischen Schwankungen über die ganze Erde relativ klein. Jährlich steigt die $CO_2$-Konzentration im weltweiten Mittel um zirka 2 ppm an, was einer relativen Konzentrationszunahme von zirka 0.5% entspricht. Auch die Methankonzentration steigt an. Diese jährliche Konzentrationszunahme ist im Wesentlichen auf die jährliche Zunahme der Menge der verbrannten fossilen Brennstoffe zurückzuführen. Jährlich werden durch solche Verbrennungen rund 180 Mio. Tonnen $CO_2$ mehr als im Vorjahr freigesetzt. Politische Kräfte werden immer stärker, die nach einer Reduktion des $CO_2$-Ausstosses rufen, und es werden ehrgeizige Ziele gesetzt, nach denen in den kommenden 5 bis 10 Jahren der $CO_2$-Ausstoss weltweit um bis zu 20% gesenkt werden soll. Gleichzeitig wird aber ein stetig steigender Energiekonsum beobachtet, und in Schwellenländern, die sich in einer Transformationsphase von einer Agrargesellschaft in eine Industrie- und Dienstleistungsgesellschaft befinden, ist dieser Anstieg sogar sehr beträchtlich. Vor diesem Hintergrund erscheint eine Stabilisierung des $CO_2$-Ausstosses wenig wahrscheinlich und eine Reduktion auf einen tieferen Gesamtwert erst recht illusorisch. Der absolut grösste Teil der Energieversorgung unserer Zivilisation basiert auf der Verbrennung fossiler Brennstoffe und es sind bisher keine wirklich ernst zu nehmenden Alternativen in Sicht, welche diese gewaltigen Energiebeträge aus Öl und Gas ersetzten könnten. Die Extrapolation der ablaufenden Entwicklung lässt deshalb befürchten, dass der $CO_2$-Ausstoss weiterhin unablässig ansteigen wird, mit all den mittel- und langfristig gravierenden Folgen. Die Schäden infolge eines einsetzenden Klimawandels sind gigantisch.

Der $CO_2$-Ausstoss ist deshalb allein für sich gesehen ein sehr ernstes Problem für die menschliche Zivilisation. Die mit ihm gekoppelte andere Problematik ist die Energieversorgung der menschlichen Zivilisation, die ebenfalls sehr gravierend ist. Denn es ist die Energieversorgung wie sie heute sichergestellt wird, welche ja gerade die $CO_2$-Problematik heraufbeschwört und zusehends verschärft. Und zahlreiche Nationen mit grossen Populationen, die gerade daran sind, auf breiterer Ebene in das industrielle Zeitalter einzutreten, entwickeln einen enormen Energiebedarf. Könnte man nach und nach oder sogar sehr rasch auf eine alternative Energieversorgung umstellen, welche $CO_2$-neutral oder gar $CO_2$-negativ wäre, so könnten zwei derzeit unauflöslich miteinander gekoppelte Kardinalprobleme der menschlichen Zivilisation mit einem Schlag gelöst werden.

Bisherige $CO_2$-Problemlösungsansätze beinhalten unter anderem das sogenannte "Carbon Capture and Storage"-Verfahren (CCS-Verfahren). Bei der Verbrennung von fossilen Brennstoffen in Kraftwerken wird das $CO_2$ eingefangen (captured), das heisst aus den Abgasen herausgewaschen und hernach komprimiert oder verflüssigt. Das $CO_2$ wird dann in geeignete geologische Formationen gepumpt, etwa in alte Öl- und Gaslagerstätten oder in poröses Gestein. Um einen weiteren Anstieg von $CO_2$ in der Atmosphäre zu vermeiden, müsste man Hunderte Millionen von Tonnen von $CO_2$ jährlich so einlagern, und das für viele Jahre, was eine fast unvorstellbare Aufgabe wäre. Wegen dieser enormen Mengen ist auch die Machbarkeit von sämtlichen solchen chemischen Ansätzen - ausserhalb von Labor- und Pilotanlage-Versuchen - unrealistisch für eine echte Lösung der $CO_2$-Problematik. Für jede Tonne eingespartem $CO_2$ wird heute wohl ein Zertifikat um die 20 USD gehandelt. Der Wirkungsgrad der Kraftwerke würde wegen der Anwendung dieser Methoden um ca. 10% herabgesetzt. Dazu kommen die Risiken, die mit einem Lagern von so grossen Gasmengen verbunden wären. Diese sind beträchtlich und es müsste mit dem Auftreten von katastrophalen "blow outs" gerechnet werden. Eine solche Katastrophe ereignete sich 1986 in Kamerun, als im Gebiet des Nyos Sees plötzlich etwa 1,7 Mio. Tonnen $CO_2$ freigesetzt wurden. Da $CO_2$ schwerer ist als Luft, erstickten im Umkreis von 20 km über 1700 Menschen! Fazit: Bei sämtlichen bis jetzt vorgeschlagenen und diskutierten konventionellen Methoden ist es nicht einmal bei optimistischer Prognose vorstellbar, dass sie über den Pilotversuch hinauskommen und einen substantiellen Beitrag zur Reduktion des $CO_2$-Ausstosses liefern.

Die Lösung des grundlegenden Problems, nämlich eine $CO_2$-neutrale Energieversorgung der zivilisierten Gesellschaften zu schaffen, ist derzeit nicht in Sicht. Die Erdölvorräte gehen zur Neige. Schon 2010 wird ein Drittel der Maisproduktion für die Ethanolproduktion benötigt werden, mit weitreichenden Folgen für die weltweite Nahrungsmittelversorgung. In der Europäischen Union will man bis ins Jahr 2020 den Energiebedarf des Verkehrssektors zu 10% mit Biotreibstoffen decken. Fahren versus essen wird zum schrecklichen Zielkonflikt.

Das Gleichgewicht von $CO_2$-Aufnahme und $CO_2$-Abgabe in die Atmosphäre hatte wohl über einige Jahrtausende Bestand, doch mit der Industrialisierung und der damit einhergehenden rasanten und exponentiellen Zunahme der Verbrennung fossiler Brennstoffe seit etwa 150 Jahren begann sich dieses Gleichgewicht zu verschieben. Heute (2008 AD) werden zirka 10 Milliarden Tonnen fossile Brennstoffe, unter anderem Erdöl, jährlich aus fossilen Quellen genutzt. Der Erdölverbrauch steigt auch heute noch jährlich um zirka 0,5 Prozent an, wenngleich das Maximum der bekannten Ölvorräte bereits überschritten zu sein scheint. Da das Wachstum der Vegetation Kohlendioxid durch die Photosynthese bindet, erlaubt das Kyoto-Protokoll Ländern mit grossen Wald- und anderen Vegetationsgebieten, einen gewissen Anteil ihrer $CO_2$-Emissionen mit den grossen $CO_2$-Aufnahmen ihrer Vegetation zu verrechnen, um so ihren Netto $CO_2$-Emissionsgrad zu senken. Auf der Basis dieser Verrechnungsmöglichkeit entstand ein internationaler "$CO_2$-Emissionsmarkt", von dem man sich erhofft, dass er lenkend wirkt und letztlich zu einer kosteneffektiven $CO_2$-Emissionsreduktion führen sollte. Es handelt sich gewissermassen um ein Bonus-Malus-System.

Aus D1: US 2002/0111527 wurde die Injektion von verflüssigter Biomasse in poröse Gesteinsformationen bekannt. Erstens handelt aber stets um verflüssigte Biomasse und zweitens wird die Biomasse nicht in einem genau vorbekannten, gegenüber dem Rest der Umwelt völlig isolierten Volumen untergebracht. Vielmehr presst man die verflüssige Biomasse in Gesteinsformationen. Die D2 (EP 0' 320 393 A1) andrerseits befasst sich mit dem Deponieren von toxischen Industrieabfällen. Diese werden in eine Salzkaverne geschüttet. Damit die toxischen Industrieabfälle nicht mit der Salzsole in Kontakt kommen, wird ein flüssiger Dichtungs-Stopfen in Form einer halogenisierten Lösung, zum Beispiel eine Chlorlösung von einem höheren spezifischen Gewicht von 1,3 bis 1,7 eingefüllt, welche dann eine gesonderte Sole unter der Salzsole bildet. Nur in diese Grundsole wird der Abfall eingebracht. In dem Mass wie Abfälle eingebracht werden, wird die Grundsole verdrängt und wächst nach oben. Das Salzwasser wird oben abgenommen und der Dichtungsstopfen wird nachgefüllt, damit es nie zu einem Kontakt der Abfälle mit dem Salzwasser kommt. (siehe Spalte 4, Zeile 6-9 sowie Zeile 31 bis 35). Diese Methode hat jedoch nicht $CO_2$-Reduktion oder eine Methangas-Reduktion in der Atmosphäre zum Ziel, noch ein Gewinnen von Biogas.

Eingedenk der oben erwähnten Ausgangslage ist es die Aufgabe der vorliegenden Erfindung, einen technisch und industriell realistischen und somit gangbaren Weg aufzuzeigen, um aus dieser fatalen Sackgasse des weiterhin steigenden Bedarfs für die Verbrennung fossiler Energieträger und des damit verbundenen $CO_2$-Anstiegs und auch Methan-Anstiegs in der Erdatmosphäre herauszukommen. Eine zweite, untrennbar damit verbundene Aufgabe besteht auch darin, einen alternativen erneuerbaren Energieträger im grossen Massstab zur Verfügung zu stellen, der die Treibhausgas-Problematik löst. Die spezifische Gesamtaufgabe der Erfindung besteht darin, ein technisches Verfahren und eine Anlage zur $CO_2$- und/oder Methangas-Reduktion in der Atmosphäre und/oder zur Gewinnung von Biogas anzugeben, sowie auch Verwendungen der Anlage aufzuzeigen.

Diese Aufgabe wird gelöst durch drei durch die Zielsetzung unterscheidbare Verfahren, welche alle durch eine gemeinsame erfinderische Idee untrennbar verbunden sind, jedoch verschiedene Ausgestaltungen des Verfahrens darstellen:

Zum ersten wird die Aufgabe gelöst von einem Verfahren zur $CO_2$- und/oder Methangas-Reduktion in der Atmosphäre, bei welchem Biomasse einer Verrottung entzogen wird, indem sie in einer unterirdischen, vom Grundwasser isolierten Kavernen anaerob eingelagert wird, sodass der organisch darin gebundene Kohlenstoff als $CO_2$-Äquivalent dauerhaft in der Kaverne eingeschlossen bleibt.

Zum Zweiten wird die Aufgabe gelöst von einem Verfahren zur Gewinnung von Biogas, bei welchem Biomasse in einer unterirdischen, vom Grundwasser isolierten und als Fermenter gestalteten Kaverne gesammelt und anaerob eingelagert wird, wobei ein Anteil durch Vergärung zu Biogas umgewandelt und abgezogen wird.

Zum Dritten wird die Aufgabe gelöst von einem Verfahren zur $CO_2$- und/oder Methangas-Reduktion in der Atmosphäre, bei welchem Biomasse einer Verrottung entzogen wird, indem sie in einer vom Grundwasser isolierten Kaverne oder einem Behälter (1,21) von wenigstens 1000m$^3$ Volumen anaerob eingelagert wird, sodass der organisch darin gebundene Kohlenstoff als $CO_2$-Äquivalent dauerhaft in der Kaverne (1,21) eingeschlossen bleibt.

Weiter wird die Aufgabe gelöst von einer Anlage zum Betreiben des Verfahrens nach einem dieser Ansprüche, die sich dadurch auszeichnet, dass sie einschliesst:

a) eine gasdichte, vom Grundwasser isolierte, unter dem Erd- oder Meeresboden in stabilen Schichten liegende baumateriallos erstellte Kaverne (1,21), in Form eines gasdichten, durchgehenden Hohlraumes von wenigstens 1000 m$^3$ Volumen, welcher gegenüber dem Rest der Umwelt ein isoliertes, abgegrenztes Volumen bildet,
b) mindestens ein Zufuhrrohr (2,22) für das Einbringen von Biomasse,
c) mindestens ein Zudosiereinrichtung (3) mit Druckschleuse zum Einbringen von Biomasse bei gleichem oder unter höherem Druck als Atmosphärendruck stehendem Kavernen- oder Behälterinnern,
d) mindestens ein Abführrohr (12,23) mit Pumpe (13) zur Entnahme von Gasen aus der Kaverne (1,21) oder dem Behälter.

Die Anlage kann verschiedene Verwendungen haben, nämlich entweder

- zum Speichern von Biogas über beliebige Zeiträume sowie zum bedarfsweisen Spenden von Biogas für eine kommerzielle Nutzung, oder
- zum Verstromen des erzeugten Biogases, oder
- zur Reinigung von Gewässern und Trinkwasserreservoirs durch Entnahme von Algen und Einlagerung derselben in einer Kaverne zur anaeroben Vergärung, oder
- zur Langzeitlagerung von $CO_2$-Äquivalenten in Form von in Biomasse gebundenem Kohlenstoff über mindestens 1 Mio. Jahre zur Einleitung der Umwandlung des Kohlenstoffes zu Kohle, Öl und Erdgas durch Fossilisierung.

Im ersten Moment erscheint dieser erfindungsgemässe Ansatz wie oben definiert wenig spektakulär. Umso überzeugender aber erweist er sich, sobald die gigantischen anfallenden Massenbeträge an Biomasse ins Kalkül gezogen werden, die erst durch den Einsatz von Kavernen riesiger Volumina überhaupt umsetzbar werden. Wenn man bei Abfall und Reststoffen bloss an Küchenabfälle und Reststoffe aus der Verarbeitung von Holz und landwirtschaftlichen Produkten denkt, so ist das Potenzial freilich beschränkt und bietet keine namhaften Beiträge. Wenn aber bedacht wird, wieviel Biomasse jährlich in der freien Natur verrottet, und man auch nur einen Teil davon effizient nutzen könnte, so ändert sich das Bild dramatisch. Durch die erfindungsgemässe Nutzung von riesigen gasdichten und baumateriallos erstellten Kavernen lassen sich diese riesigen Mengen gezielt und intelligent nutzen, mit den damit schlagenden Vorteilen, nämlich erstens einer damit verbundenen substantiellen Minderung des $CO_2$-Ausstosses in die Atmosphäre, und zweitens der Bereitstellung entsprechend grosser Mengen an Biogas für die kommerzielle Nutzung.

Die Erfindung sowie die Gedankengänge, auf denen sie basiert, werden im Folgenden dargelegt, und dazu wird auf die Zeichnungen verwiesen.
Es zeigt:

Figur 1: Eine schematische Darstellung des Biomasse-Kohlenstoff-Kreislaufs unseres Planeten mit dem erfindungsgemässen Entzug einer Teilmenge der Biomasse und des darin gebundenen Kohlenstoffs zur Bewahrung vor einer aeroben Verrottung in der Natur;

Figur 2: Eine schematische Darstellung einer Biomassen-Salzkaverne auf dem Festland zur Reduktion der Treibhausgase $CO_2$ und Methan in der Atmosphäre und zur Biogas-Erzeugung sowie zur bleibenden Kohlenstoff-Einlagerung zwecks Einleitung der Fossilisation;

Figur 3: Eine schematische Darstellung einer off-shore Biomassen-Kaverne zur Reduktion der Treibhausgase $CO_2$ und Methan in der Atmosphäre und zur Biogas-Erzeugung durch Einlagerung von Biomasse, namentlich von Algen, sowie zur bleibenden Kohlenstoff-Einlagerung zwecks Einleitung der Fossilisation;

Figur 4: Die Massenbilanz des Verfahrens, schematisch dargestellt anhand einer Tonne Biomasse, die dem natürlichen Verrottungsprozess entzogen wird.

Das Binden (capture) von $CO_2$ aus der Atmosphäre ist seit Milliarden von Jahren genial gelöst. Mittels Sonnenenergie in der Photosynthese wird nämlich $CO_2$ in der Biomasse gebunden:

$$6\ CO_2 + 6\ H_2O + Licht + in\ Verbindung\ mit\ Chlorophyll = 6\ O_2 + C_6H_{12}O_6$$

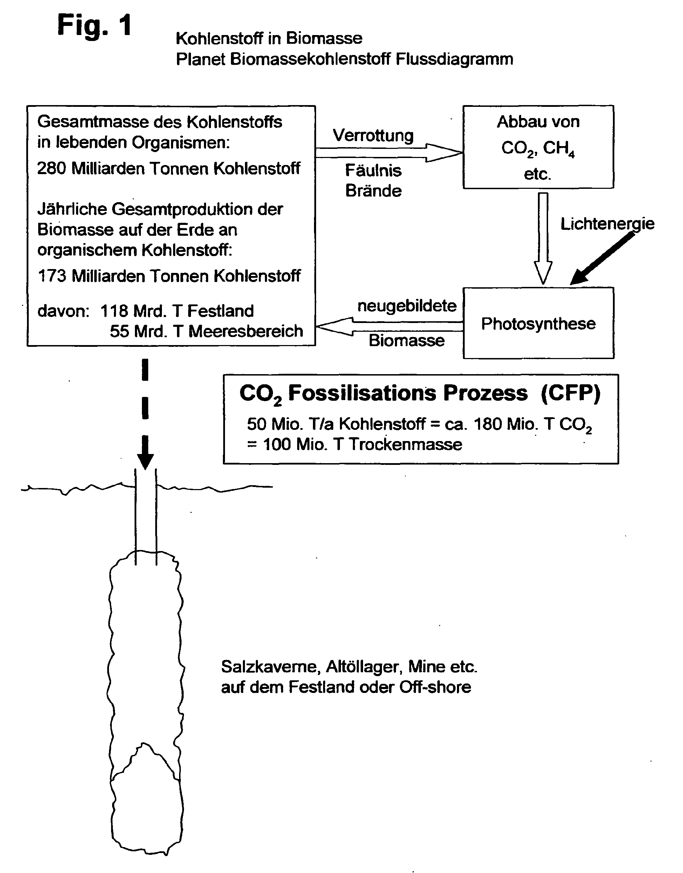

Die Figur 1 zeigt diesen Prozess schematisch auf. Von ausserhalb der Erde kommt bloss die Sonnenenergie bzw. Lichtenergie hinzu, welche den ganzen Prozess auf der Erde und in der Atmosphäre am Laufen hält. Mit der Photosynthese wird ein Wassermolekül durch zwei Lichtquanten aus der Sonnenergie gespalten zu Wasserstoff und Sauerstoff. Der Wasserstoff reduziert $CO_2$ zu organischen Kohlenstoffverbindungen als wichtigsten Bestandteil der Biomasse. Pflanzen und Algen wirken als eigentliche Biokonzentratoren, welche aus ca. 380 ppm $CO_2$ Konzentrationen in der Atmosphäre Kohlenstoff in Form von organischen Verbindungen in der Biomasse einlagern, mit einem Anteil von Kohlenstoff in den Algen von bis zu ca. 50 % ihrer trockenen Biomasse. Diese Konzentrations-Anreicherung beträgt im Vergleich zu den 380ppm in der Atmosphäre mehr als das Tausendfache! Der Prozess läuft bei Raumtemperatur ab, ohne jegliche komplizierte künstliche Technologien, und man kann zu Recht sagen: Es handelt sich um ein Wunder der Natur. Jeder Versuch, diesen natürlichen Weg des Einbindens von $CO_2$ nachzuahmen ist mit unseren technologischen Mitteln nicht einmal annähernd energieeffizient erreichbar. Eine Tonne Kohlenstoff (carbon) in Biomasse gespeichert korrespondiert mit 3,6 Tonnen gebundenem $CO_2$, was sich aus der Stöchiometrie und den Atomgewichten ergibt:

$$C + O_2 \rightarrow CO_2$$

$$12Gr. + 2 \times 16Gr. = 44Gr.$$

Aus 12 Gramm Kohlenstoff werden also 44 Gramm $CO_2$. Das ergibt einen Faktor 3.66, denn 12 x 3.66 = 44 Gramm, oder im Folgenden vereinfacht auf den Faktor 3.6. Aus 50 Tonnen gebundenem Kohlenstoff werden also: 50 Tonnen C x Faktor 3.6 = 180 Tonnen $CO_2$. Die jährliche Gesamtproduktion der Biomasse auf der Erde an organischem Kohlenstoff beträgt etwa 173 Milliarden Tonnen, davon entstehen etwa 118 Milliarden Tonnen auf dem Festland und etwa 55 Milliarden Tonnen in den Ozeanen. Etwa 10 Mia. Tonnen Kohlenstoff verbrennt die Menschheit alljährlich, Tendenz steigend, während nach Schätzungen gerundet etwa 200 Mia. Tonnen Kohlenstoff aus Biomasse in der freien Natur verrotten (das heisst so viel wie produziert wird verrottet hernach laufend), also etwa das Zwanzigfache der verbrannten Menge. Die folgende Rechnung ist nun aufschlussreich, und sie begründet die im unteren Teil des Schemas dargestellte Grundidee der Erfindung, nämlich das Abzweigen von Biomasse zwecks einer anaeroben isolierten Vergärung in einer gasdichten unterirdischen Kaverne: 1 Mio. Tonnen Biomasse reduziert sich auf ca. 0.5 Mio. Tonnen trockene Biomasse. Diese wiederum enthält 50% bis 60% biologisch gebundenen Kohlenstoff, also ca. 0.25 bis 0.30 Mio. Tonnen Kohlenstoff. Multipliziert man mit dem Faktor 3.6, so erkennt man, dass daraus ca. 0.90 bis 1.08 Mio. Tonnen $CO_2$ entstehen. Vereinfacht ausgedrückt entsteht aus 1 Tonne Biomasse entsteht etwa 1 Tonne $CO_2$! Wenn Biomasse an der Luft verrottet, wie sie das ja in der Natur weltweit in gigantischen Mengen tut, so entsteht daraus auf die Masse bezogen etwa 50% $CO_2$ und 50% Biogas bzw. Methan. Vergärt die Biomasse hingegen anaerob, so entsteht daraus nur etwa 30% $CO_2$, dafür aber 70% Biogas.

Aufgrund all dieser Erkenntnisse verfolgt die vorliegende Erfindung im Wesentlichen eine Multigas-Strategie, nämlich im grossen Stil erstens den $CO_2$-Ausstoss und $CH_4$-Ausstoss in die Erdatmosphäre zu reduzieren, und zweitens aus der Biomasse gleichzeitig nutzbares Methan zu erzeugen. Eine Verrottung von Biomasse zu klimaschädigendem $CO_2$ und Methan wird verhindert. Es wird dazu nicht mit einer technologischen "Brechstange" gegen die Natur vorgegangen, sondern es werden konsequent die existierenden Rhythmen der Naturprozesse genutzt. Es wird ein indirekter, aber dafür sehr logischer Weg gewählt: Anstatt nämlich jährlich 180 Mio. Tonnen gasförmiges $CO_2$ aufwändig einzufangen und mit einem erheblichen Gefahrenpotential lagern zu wollen, was in diesem Grössenmassstab ohnehin nicht industriell realisierbar ist, wird nun angestrebt, das biologische $CO_2$-Äquivalent von 180 Mio. Tonnen gasförmigem $CO_2$ in Form von Biomasse "einzufangen". Hierzu müssen also etwa 50 Mio. Tonnen Kohlenstoff in Biomasse gebunden vor einem $CO_2$-Ausstoss in die Atmosphäre bewahrt werden, denn 50 Mio. T C x 3.6 = 180 Mio. T $CO_2$. Entzieht man der Natur also möglichst viel Biomasse, um sie gezielt vor einer aeroben Verrottung zu bewahren, und führt sie einer anaeroben Vergärung zu, im Rahmen welcher das entstehende $CO_2$ zurückgehalten werden kann, also nicht in die Atmosphäre gelangt, so lassen sich auf diese Weise sehr erhebliche Reduktionen des weltweiten $CO_2$-Ausstosses in die Atmosphäre erreichen. Berechnungen zeigen, dass nur schon die Bewahrung von 50 Mio. Tonnen C in Biomasse vor einer aeroben Verrottung und Zurückhaltung des anaerob entstehenden $CO_2$-Gases genügt, um den weiteren Anstieg der $CO_2$-Konzentration in der Erdatmosphäre zu stoppen, welcher derzeit ca. 0.5% beträgt. Wird noch mehr Biomasse der natürlichen aeroben Verrottung entzogen, so lässt sich die $CO_2$-Konzentration sogar wesentlich senken, auf Niveaus wie sie vor Jahrzehnten vorherrschten.

Das überraschende Moment der Erfindung ist, dass dieses Bewahren und Isolieren von solch grossen Mengen von Biomasse für eine anaerobe Vergärung und gleichzeitig die Zurückhaltung des entstehenden $CO_2$-Gases technisch ohne Weiteres machbar ist. Die Biomasse (Holz, Abfallholz, abgefallene Blätter, landwirtschaftlicher Abfall, Grünabfall, Gülle, Küchenabfall, Tierkadaver, etc.) wird hierfür in gasdichten, vom Grundwasser isolierten, sauerstoff-freien Kavernen eingelagert und dort einer anaeroben Vergärung ausgesetzt. Dabei entsteht nebst Methan (70%) auch $CO_2$, aber nur zu 30%. Das so erzeugbare Biogas kann zu Bio-Methangas gereinigt werden, indem es von $CO_2$ befreit wird. Mit Aktivkohle kann ausserdem der Schwefel aus dem Biogas entfernt werden. Lösungen für diese Veredelung von Biogas sind im Stand der Technik vorhanden. Schliesslich kann Biogas auch mitsamt dem darin enthaltenen $CO_2$ verstromt werden, was speziell etwa für die dritte Welt interessant ist.

Wenn man den gegenwärtigen jährlichen 0.5% Anstieg der $CO_2$-Konzentration in der Erdatmosphäre stoppen will, so muss man wie erwähnt jährlich 50 Mio. Tonnen Kohlenstoff in Biomasse gebunden in Kavernen einlagern. Hierzu benötigt man etwa 100 Kavernen mit je einem Fassungsvermögen von 1 Mio. m$^3$. Jede solche Kaverne kann mehrmals nachgefüllt werden, denn die laufende Vergärung in der Kaverne reduziert das Volumen der darin eingelagerten Biomasse. Das Auffüllen wird fortgesetzt, bis keine nenneswerte Biogas-Produktion mehr stattfindet. Dann pumpt man das Restgas ab und reduziert den Innendruck in der Kaverne, bis diese kontrolliert kollabiert und ihren Inhalt, nämlich organisch gebundenen Kohlenstoff von bis zu 600'000 Tonnen einschliesst. Dann setzt der Fossilierungsprozess ein. Die 600'000 Tonnen C korrespondieren etwa mit einem 2.2 Mio. Tonnen $CO_2$-Aequivalent, welches $CO_2$ nicht als Treibhausgas zur Wirkung kommt und nach dem Kyoto-Protokoll als captured and stored gilt und daher als $CO_2$-Zertifikat verrechenbar und handelbar ist.

Der eigentliche Schlüssel zur Nutzung der Bioenergie liegt in der gewaltigen Grösse der Fermenter in Form von Kavernen! Eine Kaverne mit 1 Mio. m$^3$ Inhalt entspricht einem Raum eines Kubus von 100m x 100m x 100m, was im erdgeographischen Massstab ein kleines Klötzchen ist, oder es entspricht einem Bohrloch von 40m x 40m x 625m. Unterirdische, gasdichte und vom Grundwasser isolierte Kavernen von annähernd dieser Grösse gibt es bereits in grösserer Anzahl, in Deutschland allein ca. 100, wovon etwa 35 für strategische Erdölreserven und Gas benützt werden, und in den U.S.A. gibt es bereits über 1000 solche Kavernen! Solche Untergrundspeicher dienen dem Ausgleich jahreszeitlich bedingter Bedarfsschwankungen, der Abdeckung von großen Bedarfsspitzen sowie als strategische Energiereserve und Havariereserve. Sie werden auch zunehmend für Spot-Handelsgeschäfte genutzt. Unter Untergrundspeicherung ist die sichere, kostengünstige und umweltverträgliche Bevorratung großer und größter Mengen an Energieträgern, flüssigen oder gasförmigen Rohstoffen oder chemischen Erzeugnissen in den natürlichen Poren und Klüften oder in künstlich geschaffenen Hohlräumen in tief liegenden geologischen Formationen zu verstehen. Gespeichert werden Erdgas, Erdöl, Luft, Kraftstoffe, Propan/Butan, chemische und petrochemische Erzeugnisse. Die gespeicherten Mengen sind nach herkömmlichen Vorstellungen riesig. So können einige hundert Millionen $m^3$ Erdgas in einer Speicheranlage bevorratet und mehrere hunderttausend oder sogar einige Millionen $m^3$ pro Stunde ausgelagert werden. Gase werden in der Regel unter sehr hohem Druck gespeichert, der häufig mehr als 100 bar beträgt und gelegentlich auch 200 bar überschreiten kann.

Eine besondere Kategorie von Kavernen, die sich optimal als Riesen-Fermenter für Biomassen aller Art anbieten, sind Salzkavernen. Salzgestein kommt unter bestimmten Bedingungen im Untergrund mit einer beachtlichen Mächtigkeit in Form von Salzschichten oder Salzstöcken vor. Diese sind meist 500 bis 1500m mächtig. Ab einer gewissen Salzmächtigkeit können darin Salzkavernen angelegt werden. Das sind mit Wasser künstlich ausgesolte, große Hohlräume. Zur Herstellung einer solchen Kaverne wird über eine verrohrte Bohrung von ca. 60cm Durchmesser und 300m Tiefe Wasser bis in jene Tiefe einzirkuliert, in der sich das Salzgestein befindet. Dort löst das Wasser das Salz und sättigt sich auf. Die dabei entstehende Salzsole mit etwa 30% Salzgehalt fließt über einen weiteren in der Bohrung befindlichen Strang nach Übertage. Dadurch können Hohlräume mit einem Volumen von mehreren 100'000$m^3$ baumateriallos entstehen. Während der Errichtung sowie während des späteren Betriebes erfolgt die Kontrolle der Hohlraumform über Sonarmessungen. Mit geomechanischen Berechnungen wird die Stabilität der so geschaffenen Hohlräume nachgewiesen. Salzkavernen sind bisher für die Speicherung von Gasen unter hohem Druck (z.B. Erdgas) aber auch von Flüssigkeiten, die gegenüber Salz und Wasser inert sind (z.B. Rohöl, Kraftstoffe, LPG), bestens geeignet. Deren Dichtheit ist durch die plastischen Eigenschaften des Salzgesteins gewährleistet.

Mit Salzkavernen können sehr hohe Ein- und Auslagerungsraten erzielt werden, womit sie sich besonders zur Spitzenbedarfsdeckung eignen. Um 1$m^3$ Hohlraum zu schaffen müssen ca. 8$m^3$ Süßwasser aufgewendet werden. Die schadlose Beseitigung der anfallenden Salzsole stellt eine wesentliche Aufgabe bei der Kavemenerrichtung dar. Ist im Binnenland eine stoffwirtschaftliche Nutzung der Sole nicht gegeben, so besteht eine weitere umweltfreundliche Möglichkeit in der Verbringung in tiefe poröse oder klüftige Gesteinsformationen, in denen sich schon Salzwasser befindet und deren Dichtheit nachgewiesen ist. Es ist auch ein druckloses Füllen von Kavernen möglich, zum Beispiel von solchen, die mit Salzsole gefüllt sind. Durch das Füllen wird die Sole durch die eingebrachte Biomasse herausgedrückt. Die Sole kann dann filtriert und/oder zentrifugiert werden und einer Nutzung zugeführt werden.

Es gibt Spezialfirmen, welche in der Aussolung von Kavernen grosse Erfahrung haben. Die Aussolungskosten betragen ca. 10 bis 20 CHF pro $m^3$. Die Erstellungskosten für eine einzelne Kaverne liegen daher in der Grössenordnung von ein paar Millionen CHF, oder weltweit gesehen für die Erstellung von 100 Kavernen wie benötigt, um den $CO_2$-Anstieg in der Atmosphäre zu stoppen, liegen die Erstellungskosten bei zirka 5 bis 10 Mia. CHF. Damit ist die technische Machbarkeit für das Erstellen der benötigten mächtigen Kavernen wie auch die ökonomische Machbarkeit ausser Frage gestellt.

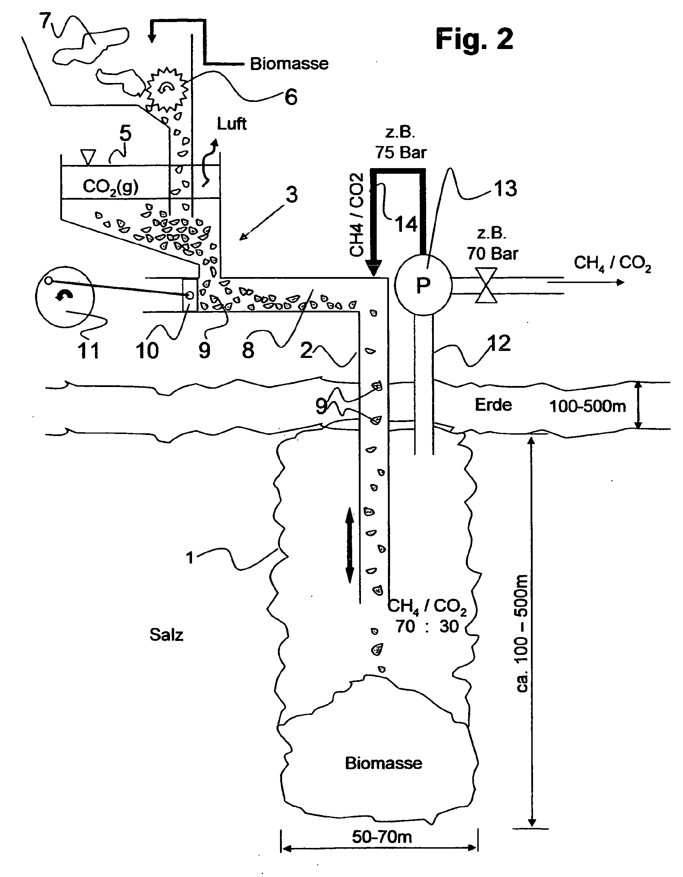

Die Anlage zum Betrieb des vorgeschlagenen Verfahrens besteht also aus einer gasdichten, vom Grundwasser isolierten Kaverne 1, wie in Figur 2 gezeigt, sei dieses eine bereits bestehende geeignete Kaverne, oder aber eine eigens zum Betrieb des Verfahrens hergestellte, ausgesolte und somit baumateriallose Kaverne, oder eine mit irgendeinem Baumaterial überirdisch oder unterirdisch erstellte Kaverne oder eine bestehende Salzkaverne in einer Salzformation. Die Kaverne 1 weist dann einen Durchmesser von in der Regel 20-100m auf und eine Höhe von 100-600m. Von oben ist diese Kaverne über mindestens ein druckfestes Rohr 2 von zum Beispiel 0.60m 0 zugänglich, um durch dieses Rohr Biomasse in die Kaverne zu pumpen. Ein weiteres Rohr 12 von ähnlichem Durchmesser enthält eine Anzahl weiterer Rohre von zum Beispiel 2" 0 oder grösser, um Gas und/oder Flüssigkeiten in die Kaverne 1 eintragen oder aus ihr austragen zu können. Eine Zudosiereinrichtung 3 mit Druckschleuse erlaubt es, Biomasse gleich welcher Art in die vorzugsweise - jedoch nicht zwingend - unter einem gegenüber dem Atmosphärendruck erhöhten Druck stehende Kaverne 1 zu befördern. Im gezeigten Beispiel schliesst diese Zudosiereinrichtung 3 eine Einschüttgosse 4 auf, in welcher ein gasförmiger $CO_2$-See 5 aufrechterhalten wird, um bestmöglich einen Sauerstoff-Eintrag in die Kaverne 1 zu unterbinden. $CO_2$ ist mit einer Dichte von 1977 kg/$m^3$ unter Normbedingungen (1013 mbar, 0 °C) etwa 1,5 mal schwerer als Luft und bleibt daher stets unten. Der Zudosiereinrichtung 3 ist eine Schredderanlage 6 vorgeschaltet, welche die angelieferte Biomasse 7 zu geeignet grossen Einheiten zerkleinert. Die von der Schredderanlage 6 in die Einschüttgosse 4 fallende Biomasse 7 trägt die darin enthaltene Luft nach oben ausgetragen und die Biomasse 7 gelangt schliesslich in die Zudosiereinrichtung 3 mit Druckschleuse. Die Zudosiereinrichtung 3 wird zum Beispiel von einer Feststoffpumpe mit von einem Kurbeltrieb 11 angetriebenem Kolben 10 oder von einer hydraulischen Membranpumpe gebildet. Solche Festkörperpumpen existieren bereits in der Mining-Industrie und sie können etwa 1'000 $m^3$ Feststoffe gegen 250 bar Druck fördern. Im Zylinder 8 wird die geschredderte und nun weitgehend sauerstoff-freie Biomasse 9 mit Hilfe der Feststoffpumpe gegen den Innendruck in der Kaverne 1 in dieselbe befördert. Das Füllen einer solchen Kaverne kann auch pneumatisch erfolgen, indem die Kaverne mit Biomasse vollgepumpt wird oder diese chargenweise über beispielsweise eine Doppelschleuse eingepresst wird. Die Biomasse fällt durch das Rohr 2 der Verrohrung in das Innere der Kaverne 1. Im Innern der Kaverne 1 wird ein Druck von 20 bis 70bar aufrechterhalten, das heisst dieser Druck bildet sich infolge der einsetzenden Vergärung bald von selbst. Durch die anaerobe Vergärung entsteht aus dem in der Biomasse eingelagerten Kohlenstoff C jeweils 70% Biogas und 30% $CO_2$. Über das Rohr 12 und die Pumpe 13 wird das Biogas und $CO_2$ aus der Kaverne 1 entnommen und einer kommerziellen Nutzung zugeführt. Entweder wird das Biogas nach Standardmethoden zu Methan gereinigt oder wird direkt mit dem $CO_2$ in Gasmotoren verstromt. Ab der Pumpe 13 kann auch Gas über ein Rücklaufrohr 14 in die Kaverne 1 gepumpt werden.

Es ist auch möglich, eine Salzkaverne, die noch voll Salzsole ist, sogleich für das Einbringen von Biomasse zu nutzen und damit die Salzsole nach oben zu verdrängen und nach und nach abzupumpen. In gleicher Weise kann auch eine bereits als Erdgaslager verwendete Kaverne genutzt werden, in welche die Biomasse eingebracht wird und damit das Erdgas nach oben verdrängt und abgepumpt wird. Damit lässt sich, wenn solche Kavernen vorliegen, wertvolle Zeit einsparen und das Verfahren lässt sich sehr rasch realisieren. Wird eine Kaverne einige Jahre betrieben und ist dann die Biogasbildung erschöpft, das heisst nach Füllung der Kaverne 1 und Abschluss des Vergärungsprozesses, so kann der Druck in der Kaverne durch Entnahme von Gas soweit reduziert werden, dass die Kaverne schliesslich mit Salzwasser gefüllt wird und hierbei die Biomasse dicht umschliesst. In einer weiteren erdgeschichtlichen Phase setzt dann die Fossilisierung des in der Biomasse gebundenen Kohlenstoffes ein und es bildet sich über grosse Zeiträume Kohle, Erdöl und Erdgas. Zudem wird das $CO_2$-Äquivalent des eingelagerten Kohlenstoffes der Atmosphäre erspart.

Im Folgenden wird der Betrieb von solchen Kavernen am Beispiel eines Szenarios dargestellt, wie es in der kleinen Schweiz, einem Land mit 7.5 Mio. Einwohnern und einer Fläche von 41'000km$^2$ technisch ohne Weiteres möglich ist: Die nachwachsende Menge von Holz beträgt in der Schweiz ca. 10 Mio. Tonnen pro Jahr, was ebenfalls ein Abbaupotential ist. Diese 10 Mio. Tonnen Holz beinhalten ca. 3 bis 4 Mio. Tonnen biologisch gebundenem Kohlenstoff. Weitere 1 bis 2 Mio. Tonnen Biokohlenstoff sind durch Aufsammeln von der zur Verfügung stehenden Biomasse verfügbar. Somit ist es vorstellbar, in der Schweiz jährlich bis zu 5 Mio. Tonnen biologisch gebundenen Kohlenstoff zu sammeln und anaerob in Kavernen zu lagern und zu vergären. Hierzu müsste pro Kopf der etwa 7 Mio. Personen zählenden Bevölkerung 1 Tonne Biomasse pro Jahr gesammelt und der natürlichen Verrottung entzogen werden, also insgesamt 7 Mio. Tonnen Biomasse. Der darin enthaltene ca. 2 Mio. Tonnen biologisch gebundene Kohlenstoff korrespondiert mit ca. 7.32 Mio. Tonnen emittiertem $CO_2$, wenn man diese Biomasse verbrennen oder aerob verrotten lassen würde. Diese 7.32 Mio. Tonnen $CO_2$ machen aber bereits 4% der Weltmenge an $CO_2$ aus, welche einzufangen und einzulagern (capture and storage) notwendig ist, um einen weiteren Anstieg der $CO_2$-Konzentration in der Erdatmosphäre zu verhindern und somit eine drohende gravierende Klimaveränderung mit all ihren katastrophalen Auswirkungen abzuwenden! Jährlich 7 Millionen Tonnen Biomasse in der Schweiz zu sammeln bedeutet ca. 700'000 Lkw-Ladungen, oder pro Tag landesweit ca. 2000 Lkw-Ladungen auf zum Beispiel 10 Kavernen verteilt, das heisst pro Kaverne und Tag 200 Lkw-Fahrten. Vorteilhafter aber wäre ein Eisenbahn- oder Schiffstransport der Biomasse, wo immer möglich. Aber an den obigen Zahlen erkennt man unmittelbar die Machbarkeit des Vorhabens.

Würde man in weiteren Ländern im Massstab zur Bevölkerungszahl das Gleiche tun, das heisst pro Kopf nur schon 1 Tonne Biomasse jährlich sammeln und der natürlichen Verrottung entziehen, so wäre das $CO_2$-Problem bald gelöst. Nicht nur kann ein weiterer Anstieg innert weniger Jahre verhindert werden, nein, sogar eine wesentliche Senkung der $CO_2$-Konzentration in der Atmosphäre ist mittelfristig möglich.

Der zweite eindrückliche Aspekt der Erfindung ist die im Zusammenhang mit der $CO_2$-Reduktion einhergehende mengenmässig sehr erhebliche Biogasgewinnung unter kontrollierten Bedingungen, wobei eine unmittelbare kommerzielle Nutzung dieses wertvollen Gases ermöglicht wird. Würde weltweit pro Kopf der Erdbevölkerung jährlich bloss 1 Tonne Biomasse eingesammelt und in der beschriebenen Weise einer anaeroben Vergärung zugeführt, so wären das 7 Milliarden Tonnen pro Jahr. Diese Menge enthält 2.1 Milliarden Tonnen Kohlenstoff. Und daraus entstehen durch anaerobe Fermentierung in der Kaverne 1.13 Milliarden Tonnen $CO_2$, und unter dem Strich würden der Atmosphäre 3.78 Milliarden Tonnen $CO_2$, die durch sonst durch aerobe Verrottung entstünden, minus die 1.13 Milliarden Tonnen $CO_2$ aus der Kaverne = 2.65 Milliarden Tonnen $CO_2$ erspart. Desweiteren entstehen in den Kavernen 0.98 Milliarden Tonnen Biogas. Das entspricht energetisch ca. 10% der gegenwärtigen Weltproduktion an fossilen Brennstoffen bzw. 16% des Gas- und Ölverbrauchs weltweit. Schliesslich verbleiben 1.05 Milliarden Tonnen Kohlenstoff, das heisst die Hälfte des total eingelagerten Kohlenstoffes in der Kaverne, und dieser Kohlenstoff wird über die nachfolgenden grossen erdgeschichtlichen Zeiträume fossilisiert. Zur Erinnerung: 200 Millionen Tonnen $CO_2$ müssten jährlich vor einer Emission in die Atmosphäre bewahrt warden, um den $CO_2$-Anstieg in der Atmosphäre zu stoppen! Die Welt könnte also mit diesem Verfahren ohne Weiteres das 20-fache und mehr von dem bewältigen was dringend nötig ist, um einen weiteren Anstieg der $CO_2$-Konzentration in der Atmosphäre zu stoppen. Der Anstieg der $CO_2$-Konzentration lässt sich durch blosses jährliches Einlagern von einer Tonne Biomasse pro Kopf der Erdbevölkerung stoppen! In wenig entwickelten Ländern mit üppiger Vegetation könnte das Einsammeln belohnt werden und daraus könnten breit angelegte und sinnvolle Beschäftigungsprogramme zum Segen der dortigen Bevölkerung entwickelt werden.

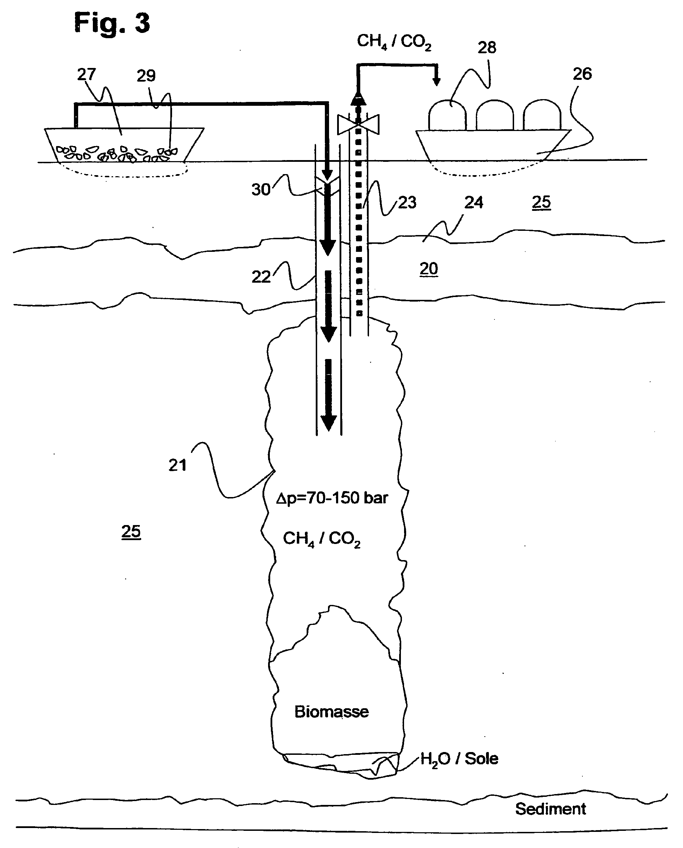

Das hier vorgestellte Verfahren erlaubt auch eine Nutzung der Meeres-Biomasse zur Reduktion des $CO_2$-Ausstosses und zur Energiegewinnung. In Figur 3 ist hierzu eine schematische Darstellung einer off-shore Biomassen-Kaverne zur $CO_2$-Reduktion in der Atmosphäre und zur Biogas-Erzeugung durch Einlagerung von Biomasse gezeigt, namentlich von Algen. Diese sind Biokonzentratoren von Dreck und entstehen mit Schmutzwasser, $CO_2$ und Licht, und sie enthalten im Trockenzustand bis zu 50% Kohlenstoff. Salzkavernen werden schon jetzt auch unter dem Meeresgrund ausgesolt. Eine solche Salzkaverne 21 lässt sich nach dem gleichen Prinzip wie schon beschrieben als gigantischer Fermenter für Meeres-Biomasse nutzen. Die Kaverne 21 wird unterhalb der wasserdichten Sedimentschicht 20 des

7

Meeresbodens 24 in einer dort vorhandenen stabilen Salzschicht 25 angelegt und nach oben bis über den Meeresspiegel verrohrt. Mindestens ein Rohr 22 dient zur Zufuhr von Biomasse. Mindestens ein Rohr 23 dient zur Entnahme von Biogas. Schiffe 27 sammeln Algen 29 aus dem oberen Schichtbereich des Meeres oder anderer Gewässer ein, indem algenhaltiges Wasser abgepumpt und in die Schiffstanks geladen wird. Für die Algenernte bestehen bereits speziell konstruierte Schiffe 27. Das Algenwasser wird hernach mittels einer Pumpe aus den Schiffen 27 abgepumpt. Die Algen werden abgeschieden und über eine Doppelschleuse 30 in die als Fermenter wirkende Kaverne 21 befördert. Durch die anaerobe Vergärung entsteht Biogas und Kohlendioxid und der Druck steigt in der Kaverne 21 an. Das Biogas wird einer kommerziellen Nutzung zugeführt, indem es in Schiffe 26 mit Gastanks 28 verladen wird und hernach distribuiert wird. Während der Trocknung von Algen beginnt bereits deren Vergärung, deswegen müsste man mit den Algen direkt in einen Fermenter, und zwar in einen grossen. Bisher lohnte sich das nicht, denn der hohe Wassergehalt machte die vergleichsweise kleinen Fermenter uneffizient. Das ändert sich hingegen beim Einsatz von gigantischen Fermentern in Form ganzer Kavernen, bei denen der Wassergehalt egal ist. 55 Mia. Tonnen Algen entstehen in den Meeren alljährlich. Das ist ein gigantisches C-Reservoir, das wie aufgezeigt erstmals intelligent angezapft werden kann.

Mit dieser Variante des Verfahrens lassen sich außerdem die enormen Algenbelastungen insbesondere im Mittelmeer vorteilhaft und effizient reduzieren. Da viele dieser Algen zudem grössere Mengen an Sulfaten, Phosphaten und anderen Salzen gebunden haben, können diese damit ebenfalls im gleichen Zug beseitigt werden. Diese Tatsache eröffnet die Möglichkeit, grosse Gewässer und Trinkwasserreservoirs nachhaltig zu reinigen.

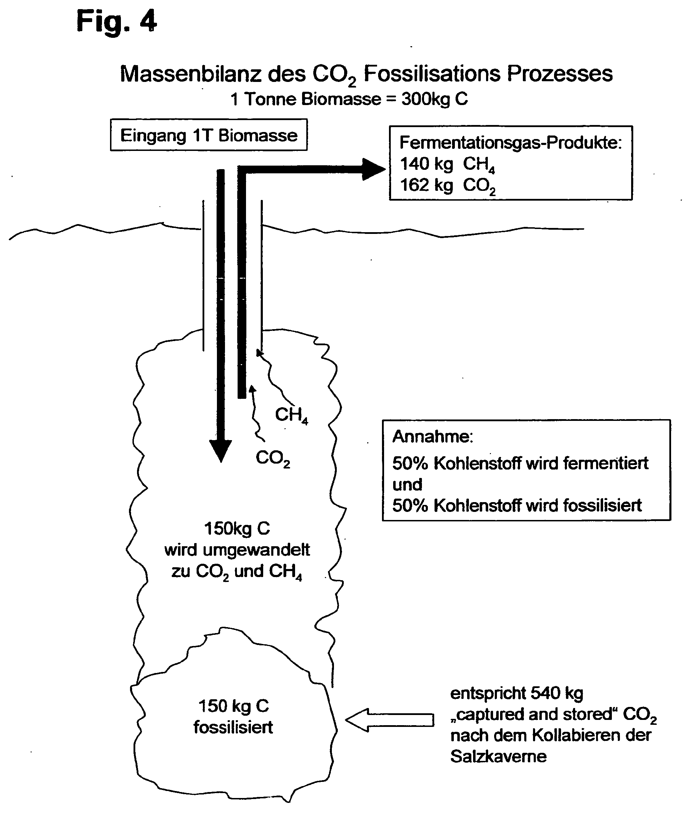

In **Figur 4** ist eine Massenbilanz des $CO_2$ Fossilisations-Prozesses gemäss dem hier vorgestellten Verfahren schematisch dargestellt. Dabei wird betrachtet, was mit einer einzelnen Tonne Biomasse passiert, welche verfahrensgemäss der natürlichen Verrottung entzogen wird. Es wird angenommen, diese enthalte 300 kg organisch gebundenen Kohlenstoff, wovon die Hälfte fermentiert wird, die andere Hälfte in den Fossilisierungsprozess übergeführt wird. Aus dieser 1 Tonne Biomasse und ihren 300kg C entstehen sodann durch eine anaerobe Vergärung ca. 140 kg Biogas bzw. Methan $CH_4$ und ca. 162 kg $CO_2$. 150 kg C verbleiben in der Kaverne. Diese dauerhaft eingelagerten 150kg Kohlenstoff entsprechen 540kg "captured and stored" $CO_2$. Dieser Kohlenstoff wird nach dem Kollabieren der Salzkaverne in den Fossilisierungsprozess übergeführt.

Es kommt nun ein weiterer sehr wichtiger Aspekt in Bezug auf die Treibhausgas-Problematik hinzu. Nach heutiger Erkenntnis sind für den Treibhausgas-Effekt vor allem drei Gase verantwortlich:

- Kohlendioxid $CO_2$ zu 75%
- Methan $CH_4$ zu 20%
- Stickstoffoxid $N_2O$ zu 5-6%

Nicht jedes Gas entfaltet aber die gleich effektive Wirkung in Bezug auf den Treibhauseffekt. Wenn man einem $CO_2$-Molekül willkürlich den Wert 1 zuordnet, so muss man dem Methan-Molekül in Bezug auf seine Wirkung als Treibhausgas den Faktor 23 zumessen, und dem Stickstoffoxid gar einen Faktor von 270 bis 310. Die Reduktion eines Methan-Ausstosses in die Atmosphäre zeigt also eine 23-mal stärkere Wirkung in Bezug auf die Treibhauswirkung als jene einer Reduktion des $CO_2$-Ausstosses. Man spricht von einem $CO_2$-Äquivalent von 23. Dieser Sachverhalt kommt dem vorliegenden Verfahren ausserordentlich entgegen und macht die Gesamtbilanz erst recht attraktiv. Man spricht deshalb von einer konsequenten Anwendung einer Multigas-Strategie. Durch das Entziehen von Biomasse aus der natürlichen aeroben Verrottung in der Natur wird entsprechend auch ein Ausstoss von Biogas bzw. Methan in die Atmosphäre verhindert, und zwar um die gleiche Masse wie $CO_2$. Aber der eingesparte Ausstoss wirkt sich 23 mal stärker aus als der gleichzeitig eingesparte Ausstoss von $CO_2$. Dieses wurde in den dargestellten Berechnungen noch gar nicht berücksichtigt, weil die ganze Treibhaus-Problematik in der Regel auf den Ausstoss von $CO_2$ reduziert wird, welches am Treibhauseffekt den Löwenanteil von 75% hat.

Neben Salzkavernen und alten Ölfeldern kommen auch alte Bergbauminen zur Verwendung als $CO_2$-Senke in Frage. Bei jeder Kaverne muss die entsprechende geologische Überprüfung auf ihre Eignung vorgenommen werden. Nur wenn diese Kavernen mit Sicherheit nicht über das Grundwasser mit der Biosphäre in Kontakt steht und die Senke weitgehend gasdicht ist und nach Möglichkeit in einem geologisch beruhigten Gebiet liegt, ist sie für das hier beschriebene Verfahren geeignet. Ist eine solche Senke gefunden, so kann deren Füllung mit Biomasse sofort begonnen werden. Mit dem erfindungsgemässen Verfahren ist die nutzbare Biomasse weder in ihrer Form noch in der chemischen Zusammensetzung eingeschränkt. Und so können über die Algen sowohl Phosphate wie auch Nitrate mit in die Senke eingespiesen werden. Gerade in den Gebieten wie der Adria, in gewissen Ostseebuchten oder in der Saragossa sind riesige Algenvorräte vorhanden, die durch ihren Abbau nicht nur eine $CO_2$ Emissionsreduktion bewirken würden, sondern zusätzlich eine Verbesserung der Wasser- und Luftqualität. Während des Füllvorganges wird man normalerweise während einer Zeit von zirka 1 bis 10 Jahren Biogas bzw. Methangas gewinnen. Ist die Biogasentwicklung jedoch weitgehend abgeschlossen, so wird man die Senke endgültig versiegeln und die Kaverne durch Druckabfall zum Einsturz bringen lassen, sodass ihr Inhalt vollständig und dauerhaft dichtend umschlossen wird.

Weitere sehr wichtige Aspekte des grundsätzlichen Verfahrens sind Folgende:

1. Die nach dem erfindungsgemässen Verfahren betriebenen Kavernen wirken als gigantische Fermenter und $CO_2$-Senken, und nur solche können einen wirklich substantiellen Beitrag an die weltweite Treibhausgas-Problematik liefern.

2. Das erfindungsgemässe Verfahren ist mit jeglicher Art Biomasse betreibbar und somit nicht eingeschränkt, weder in Bezug auf die Beschaffenheit, noch die Grösse, noch die Qualität der Biomasse, ob nass trocken, pflanzlich oder tierisch. Alles und jedes was biologisch abbaubar ist, kann in diese gigantischen Fermenter eingebracht werden.

3. Das erfindungsgemässe Verfahren ist komplett saisonunabhängig betreibbar. Fällt viel Biomasse zum Entsorgen an, so kann viel in den Fermenter befördert werden, fällt wenig an, etwa im Winter, so wird wenig bis nichts hineingefördert. Der Vergärungsprozess schreitet ohnehin voran und es wird ständig Biogas bzw. Methan erzeugt.

4. Die nach dem erfindungsgemässen Verfahren betriebenen Kavernen wirken als gigantische Lanozeitspeicher für Biogas bzw. Methan. Die Speicherung von Energie ist stets ein wichtiges Thema. Gerade elektrische Energie ist kaum in grossen Beträgen speicherbar. In den Kavernen aber können gigantische Energiemengen zu äusserst tiefen Kosten gespeichert werden. Methan kann jederzeit entnommen werden oder auch nicht. Ohne Entnahme steigt der Innendruck in der Kaverne langsam an, und er darf bis zu ca. 200 bar erreichen.

5. Das erfindungsgemässe Verfahren ermöglicht mit der Erzeugung von Biogas bzw. Methan aus nachwachsender Biomasse die Bereitstellung erneuerbarer Energie in bisher nicht bekannten Quantitäten. (1 Mio. Tonnen Biomasse in einer Kaverne mit 1 Mio. $m^3$ Inhalt erzeugt jährlich ca. 140'000 Tonnen Biogas bzw. Methan, und das wegen der laufenden Nachfüllung von Biomasse über Jahre hinweg.)

6. Das erfindungsgemässe Verfahren ist kaum geographisch gebunden und kann in allen Erdteilen umgesetzt werden, wo Vegetation vorkommt, sei es auf dem Festland, oder in Gewässern, wo Algen vorkommen, oder in den Meeren, welche ein enormes Potential aufweisen, das derzeit noch brach liegt.

7. Das erfindungsgemässe Verfahren ist kommerziell hochinteressant und daher gewerblich anwendbar, wie nachfolgend dargestellt wird.

Nachfolgend wird eine grobe Betrachtung über die Investitions- und Betriebskosten des Verfahrens angestellt. Sie zeigt, dass das Verfahren ohne jeden Zweifel gewerblich anwendbar ist, und zwar hochrentabel. Dabei wird die Annahme zugrundegelegt, man betreibe eine Kaverne von 1 Mio. $m^3$ Inhalt, und diese würde nach und nach mit 600'000 Tonnen Kohlenstoff in Biomasse gebunden gefüllt.

Der Aufwand: Die Investitionskosten bei erhärteten Erstellungskosten einer Salzkaverne von CHF 30 bis 50/$m^3$ betragen CHF 30 bis 50 Mio. Das Sammeln von Biomasse, der Antransport, das Befüllen und Betreiben kostet weitere CHF 20 bis 50 Mio., was also Gesamtkosten von CHF 50 bis 100 Mio. ergibt. Nicht berücksichtigt bei dieser Überschlagsrechnung sind:

- Allfällige Einnahmen aus kostenpflichtigem Bioabfall, wie zum Beispiel Laub, Stadtreinigungsabfall, Klärschlamm, etc.
- Allfällige stromproduzierende Infrastruktur zur Verstromung des erzeugten Methans (geschätzte Kosten CHF 5 bis 10 Mio).
- Methangasreinigungs- und Veredelungskosten (5-20% des Methanwertes).
- Optimierung des Verkaufszeitpunktes des Methans, was durch die Langzeigspeicherung bedeutsam ist. Es kann je nach Abgabezeit (Tag/Nacht - Sommer/Winter) bis zum doppelten Preis gelöst werden.

Der Ertrag: Von dem in Biomasse gebundenen Kohlenstoff von 600'000 Tonnen verbleiben 50% in der Kaverne und fossilisieren. Die verbleibenden 300'000 Tonnen würden an der Erdoberfläche durch aerobe Verrottung in der Natur $CO_2$ emittieren, nämlich 300'000 Tonnen C x 3.6 = 1.08 Mio. Tonnen $CO_2$. Pro solche eingesparte Tonne werden heute von Regierungen CHF 40 bis CHF 100 bezahlt.

Die gesamte $CO_2$-Bilanz des erfindungsgemässen Verfahrens sieht dann so aus:

| | |
|---|---|
| Zunächst verhinderter $CO_2$-Ausstoss (Fossilisation): | 1.080 Mio. T $CO_2$ |
| Minus $CO_2$-Ausstoss bei Vergärung: | 0.324 Mio. T $CO_2$ |
| Total vermiedener $CO_2$-Ausstoss: | 0.756 Mio. T $CO_2$ |

Ertragsrechnung, ohne Berücksichtigung irgendwelcher Subventionen für emeuerbare Energien oder garantierte Strompreise.

$CO_2$-Zertifikate: 756'000 T x CHF 10/T = (könnte aber auch das 10-fache sein, daher nur als Grössenordnung zu verstehen).                CHF 7.56 Mio.

(fortgesetzt)

| | |
|---|---|
| Erzeugtes Methan: 280'000 T $CH_4$ zu CHF 1000/T (Das emittierte $CO_2$ wurde schon im Aufwand berücksichtigt). | CHF 280.00 Mio. |
| Vermiedene Methanemission aus 600'000 T C in Biomasse in der freien Natur: 9.2 Mia. T $CO_2$ x CHF 10/T (600 kg C emittieren 400kg $CH_4$ x Äquivalenzfaktor 23, entspricht 9200 kg $CO_2$) | CHF 92.00 Mio. |
| Gesamtertrag | CHF 379.56 Mio. |

Dieser Gesamtbetrag ergibt sich aus den getroffenen Annahmen zur Erläuterung des Zustandekommens der Deckungsbeiträge, weist aber nicht auf die Klasse der Genauigkeit hin.

Dieser Gesamtertrag steht Gesamtkosten von minimal CHF 50 Mio. und maximal CHF 100 Mio. gegenüber. Die Zeitdauer zur Erstellung einer Anlage sollte 2 bis 3 Jahre nicht überschreiten. Weltweit könnten ohne weiteres 100 solche Anlagen oder mehr erstellt und betrieben werden. Die Verstromung des im Beispiel erzeugten Biogases bzw. Methans ergibt folgende Rechnung: 1m$^3$ Methan ergibt ca. 6 kWh elektrische Energie. Daher: 280'000 T Methan ergeben 2,352 Milliarden kWh thermische Energie. Die daraus erzeugbare elektrische Energie ist 1/3, das heisst 784 Mio. kWh, die kWh zu ca. CHF 0.10 bis 0.20 = 78 bis 156 Mio. CHF. In der Schweiz wird für Strom, der aus Biogas erzeugt wurde, ein Abnahmepreis von CHF 1.-/kWh garantiert. Dann wäre der Deckungsbeitrag aus der Verstromung sage und schreibe 783 Mio. CHF! Es ist ausserdem vorstellbar, dass Länder, welche derartige Kavernen mit Biomasse füllen, entsprechende $CO_2$-Emissionsgutschriften erhalten, die sie mit anderen Ländern, die eine hohe $CO_2$ Emissionsrate aufweisen, handeln können.

Ingesamt und sehr summarisch lässt sich abschließend Folgendes überschlagsmässig festhalten: Die 100 Kavernen, die es weltweit braucht, um den weiteren $CO_2$-Anstieg zu stoppen, kosten 20 Mia. CHF, aber diese Anlagen bringen pro Jahr 50 Mia. CHF an Erlösen. Die erzeugte und nutzbare Methanmenge würde bei diesem Bestand an Anlagen 50% von sämtlichen fossilen Brennstoffen decken, die derzeit zum Transport weltweit verbraucht werden. Methan kann auch zum Heizen oder zum Verstromen eingesetzt werden. In diesem Fall wird eine Infrastruktur zur Erzeugung von elektrischem Strom aus Methangas nach dem Stand der Technik in die Anlage integriert. Der aus Methangas bzw. Biogas erzeugte elektrische Strom wird sogar subventioniert und in der Schweiz wie auch anderswo zu garantierten Preisen von 1 CHF/kWh ins Netz eingespeist. Das ist etwa das 20-fache des Nachtstrompreises. Ingesamt löst dieses technische Verfahren wie hier vorgestellt nicht nur ein Menschheits-Problem, sondern gleichzeitig kann damit auch Geld verdient werden. Damit ist auch die gewerbliche Anwendbarkeit des Verfahrens nachweislich gegeben, und zwar weit über alle Erwartungen hinaus.

## Patentansprüche

1.   Verfahren zur Gewinnung von Biogas, bei welchem Biomasse aus Standorten in der Atmosphäre einer aeroben Vergärung entzogen wird, indem sie einer unterirdischen anaeroben Vergärung zugeführt wird, *dadurch gekennzeichnet,* dass sie als Feststoffe durch ein gasförmiges $CO_2$Bad (5) oder $H_2O$-Bad mittels einer Festkörperpumpe (11) als Druckschleuse in eine unterirdische, vom Grundwasser isolierte und als Fermenter gestaltete Kaverne (1,21) als gasdichter, durchgehender Hohlraum von wenigstens 1000m$^3$ Volumen, welche gegenüber dem Rest der Umwelt ein isoliertes Volumen bildet, gefördert wird, wobei die Biomasse daringesammelt und anaerob eingelagert wird und ein Biomassenanteil durch Vergärung zu Biogas umgewandelt und abgezogen wird, und der restliche Biomassenanteil mit seinem organisch gebundenen Kohlenstoff als $CO_2$-Äquivalent in der Kaverne dauerhaft eingeschlossen bleibt, sodass im Vergleich zur Belassung dieser Biomasse an den Standorten in der Atmosphäre eine $CO_2$- und/oder Methan-Reduktion in der Atmosphäre bewirkt wird.

2.   Verfahren nach Anspruch 1, **dadurch gekennzeichnet, dass** die Kaverne (1,21) eine durch Aussolung eigens für den Betrieb des Verfahrens baumateriallos erstellte oder zu früheren Zeiten für andere Zwecke gewonnene Salzkaverne ist, welche unter dem Erd- oder Meeresboden liegt.

3.   Verfahren nach Anspruch 1, **dadurch gekennzeichnet, dass** bei einer Salzkaverne, die noch voll Salzsole ist, die Salzsole durch Beaufschlagen des Solespiegels nach oben verdrängt und abgepumpt wird und der gewonnene Raum mit Biomasse drucklos aufgefüllt wird.

4.   Verfahren nach einem der Ansprüche 1 bis 5, **dadurch gekennzeichnet, dass** die Kaverne (1,21) eine bereits als Erdgaslager verwendete Kaverne ist, in welche die Biomasse eingebracht wird und damit das Erdgas nach oben verdrängt und abgenommen wird.

5. Verfahren nach einem der Ansprüche 1 bis 4, **dadurch gekennzeichnet, dass** zum gezielten Unterstützen von methanbildenden Prozessen für Zellulose, Lignin etc. methanproduzierende Bakterien aus Termiten und/oder Mägen von Pflanzenfressern in die Kaverne (1,21) eingebracht werden, sowie Sauerstoff unterhalb der Explosionsgrenze injiziert wird.

6. Anlage zum Betreiben des Verfahrens nach einem der Ansprüche 1 bis 5, **dadurch gekennzeichnet, dass** sie mindestens einschliesst:

   a) eine gasdichte, vom Grundwasser isolierte, unter dem Erd- oder Meeresboden in stabilen Schichten liegende, baumateriallos erstellte Kaverne (1,21) in Form eines gasdichten, durchgehenden Hohlraumes von wenigstens 1000m$^3$ Volumen, welcher gegenüber dem Rest der Umwelt ein isoliertes, abgegrenztes Volumen bildet,
   b) mindestens ein Zufuhrrohr (2,22) für das Einbringen von Biomasse,
   c) mindestens ein Zudosiereinrichtung (3) mit Druckschleuse zum Einbringen von Biomasse bei gleichem oder unter höherem Druck als Atmosphärendruck stehendem Kaverneninnern,
   d) mindestens ein Abführrohr (12,23) mit Pumpe (13) zur Entnahme von Gasen aus der Kaverne (1,21).

7. Anlage nach Anspruch 6, **dadurch gekennzeichnet, dass** die Kaverne (1,21) von einer der folgenden Arten ist:

   • eine durch Aussolung erzeugte, baumateriallos erstellte Salzkaverne, sodass sie infolge der baumateriallosen Erstellung in einer beliebigen Grösse zwischen 1000m$^3$ bis 100 Mio. m$^3$ erstell- und betreibbar ist,
   • eine natürlich vorkommende Salzkaverne in einem Salzstock ist,
   • eine natürlich vorkommende Kaverne in einer geologischen Formation,
   • eine ausgediente Miene oder eine ausgediente Öl- oder Gaslagerstätte, wobei eine Zudosiereinrichtung (3) mit Druckschleuse eine Festkörperpumpe mit hydraulischer Membrane vorhanden ist.

## Claims

1. Process for obtaining biogas wherein biomass is being withdrawn from locations in the atmosphere and hence withdrawn from an aerobic fermentation, by being supplied to an underground anaerobic fermentation, *characterized in that* the biomass is being conveyed as solids through a gaseous $CO_2$ bath (5) or $H_2O$ bath by solids pump (11) as a pressure lock into an underground, from groundwater insulated cavern which is designed as a fermenter cavity (1,21), in the form of a gas-tight, continuous cavity of at least 1000m$^3$ volume, which comprises an isolated volume against the rest of the environment, and wherein the biomass is collected and stored anaerobically and a fraction of the biomass is converted into biogas by fermentation and then withdrawn, and wherein the remaining biomass fraction with its organic bound carbon as $CO_2$ equivalent remains permanently trapped in the cavern, so that in comparison to leaving the biomass at the sites in the atmosphere, a CO2 and/or methane-reduction is effected in the atmosphere.

2. Method according to claim 1, *characterized in that* the cavern (1,21) is being created by solution mining withouth any building material specifically for the operation of the process or that the cavern is a salt cavern which was used in earlier times for the salt production and which is located under the ground or under the sea floor.

3. Method according to claim 1, *characterized in that* a salt cavern, which is still full of salt brine, the brine is being pumped out by pressing air onto the brine level and the empty space gained is being filled pressureless biomass.

4. Method according to one of claims 1 to 5, *characterized in that* the cavern is an already used cavern which is beingi used as a natural gas storage cavern (1.21), in which the biomass is being introduced in order to displaced and remove the contained gas.

5. Method according to one of claims 1 to 4, *characterized in that* for selectively supporting methanogenic processes for cellulose, lignin, etc. methane producing bacteria of termites and / or stomachs of herbivores are being introduced into the cavity (1,21), and oxygen is being injected below the explosive limit.

6. System for operating the method according to one of claims 1 to 5, *characterized in that* it includes at least:

   a) a gas-tight cavern (1,21), created without any building material, isolated from groundwater under the ground or sea floor, lying in stable layers in the form of a gas-tight, continuous cavity of at least 1000m$^3$ volume, which

forms an isolated, delimited volume compared with the rest of the environment,
b) at least one supply tube (2,22) for the introduction of biomass
c) at least one dosing device (3) with a pressure lock for the introduction of biomass at the same or at a higher pressure than the atmospheric pressure which higher pressure is standing in the cavern,
d) at least one discharge pipe (12,23) with pump (13) for removal of gases out of the cavern (1,21).

**7.** Installation according to claim 6, ***characterized in that*** the cavern (1,21) is from one of the following kinds:

• a salt cavern, generated by solution mining and established without any building materials, so that it is, due to the creation without using any building materials, is being creatable and operable in any size from $1000m^3$ to 100 million $m^3$.
• is a naturally occurring salt cavern in a salt dome,
• a cavity in a naturally occurring geological formation,
• a disused mine or a disused oil or gas reservoir,

wherein a metering device (3) with a solid state pressure lock and a solid material pump with hydraulic pump diaphragm is present.

## Revendications

**1.** Procédure pour la fabrication de gaz biologique, en cas de quel biomasse de emplacements est retiré dans l'atmosphère d'une fermentation anaérobique, en alliant celle-là à une fermentation anaérobique sous-terraine, ***caractérisé en ce que*** elle est acheminé en tant que matière solide en passant par un bain gazeux CO2 (5) ou un bain H2O parmi d'une pompe à matière solide (11) sous forme d'une écluse à pression vers une caverne sous-terraine (1,21) isolée de la nappe phréatique et sous forme d' une caverne façonnée en tant que cuve de fermentation (1,21) en forme d'un cavet dense continu ayant un volume 1000m3 minimum qui forme un volume isolé en face du reste de l'environnement, est acheminée, cependant la biomasse est ramassée et entreposé anaérobique là dedans et une partie de la biomasse est gazéifiée et enlevée en gaz biologique moyennant fermentation, et la partie restante de la biomasse restant enfermée à demeure dans la caverne , ainsi que comparé avec si on laisse cette biomasse dans les emplacements, une réduction de COS ou/et méthane est atteint dans l'atmosphère.

**2.** Procédure d'après revendication 1, ***caractérisé en ce que*** la caverne (1,21) construite par lessivage d'une caverne à sel située en dessous du sol ou du fond de la mer, spécifiquement fabriquée pour le fonctionnement de la procédure sans matériau de construction ou faite autrefois pour autres sens.

**3.** Procédure d'après revendication 1, ***caractérisé en ce que*** en cas d'une caverne à sel qui est encore pleine de saumure, la saumure est déplacée en amont et pompée à force de pressuriser le niveau de la saumure et l'espace gagné est rempli sans pression avec biomasse.

**4.** Procédure d'après une des revendications 1 à 5, ***caractérisé en ce que*** la caverne (1,21) est une caverne déjà utilisé comme dépôt de gaz dans laquelle biomasse vient insérée et ainsi le gaz naturel est déplacé vers le haut et pris.

**5.** Procédure d'après revendications 1 à 4, ***caractérisé en ce que*** des microbes qui produisent méthane consistant en termites et/ou estomacs phytophages sont introduites dans la caverne (1,21) pour ciblé soutenir des processus composant le méthane pour cellulose, lignine etc., ainsi que oxygène est engagé inferieur de la limite d'explosibilité.

**6.** Aménagement pour pratiquer la procédure d'après une des revendications 1 à 5, ***caractérisé en ce qu'***il comprend au moins:

a) une caverne (1,21) fabriquée sans matériaux de construction dense qui est isolée de la nappe phréatique et qui se trouve en dessous du solo u du fond de la mer dans des couches stables, sous forme d' un cavet dense continu de 1000m3 volume en minimum, qui, en regard du reste de l'environnement, forme un volume isolé et délimité,
b) une tube d'alimentation (2,22) du moins pour l'introduction de biomasse,
c) a minima un aménagement à dosage (3) avec écluse à pression pour l'introduction de la biomasse alors que la pression reste la même ou majeure à la pression dans l'atmosphère à l'intérieur de la caverne,
d) au moins une tube à déduction (12,23) avec pompe (13) pour la reprise de gazes de la caverne (1,21).

**7.** Aménagement d'après revendication 6, *caractérisé en ce* **que** la caverne (1,21) est une des natures suivantes:

• une caverne à sel produise sans matériaux de construction par lessivage, ainsi que en conséquence de la fabrication sans matériaux de construction dans une taille quelconque entre 1000m3 et 100 Mio. m3, elle peut être établi et pratiquée,
• une caverne à sel de présence naturelle dans un dôme salin,
• une caverne de présence naturelle dans une formation géologique,
• une mine ou un gisement à huile ou gaz ayant fait son temps, cependant un aménagement à dosage (3) avec écluse à pression et une pompe à corps solide avec membrane hydraulique existe.

# Fig. 1

Kohlenstoff in Biomasse
Planet Biomassekohlenstoff Flussdiagramm

Gesamtmasse des Kohlenstoffs
in lebenden Organismen:

280 Milliarden Tonnen Kohlenstoff

Jährliche Gesamtproduktion der
Biomasse auf der Erde an
organischem Kohlenstoff:

173 Milliarden Tonnen Kohlenstoff

davon: 118 Mrd. T Festland
55 Mrd. T Meeresbereich

Verrottung

Fäulnis
Brände

Abbau von
$CO_2$, $CH_4$
etc.

Lichtenergie

neugebildete
Biomasse

Photosynthese

**$CO_2$ Fossilisations Prozess (CFP)**

50 Mio. T/a Kohlenstoff = ca. 180 Mio. T $CO_2$
= 100 Mio. T Trockenmasse

Salzkaverne, Altöllager, Mine etc.
auf dem Festland oder Off-shore

Fig. 2

# Fig. 3

CH$_4$ / CO$_2$

27  29

28

26

30

23  24  25

22  20

21

25

$\Delta$p=70-150 bar

CH$_4$ / CO$_2$

Biomasse

H$_2$O / Sole

Sediment

# Fig. 4

## Massenbilanz des $CO_2$ Fossilisations Prozesses
### 1 Tonne Biomasse = 300kg C

Eingang 1T Biomasse

Fermentationsgas-Produkte:
140 kg $CH_4$
162 kg $CO_2$

$CH_4$

$CO_2$

150kg C
wird umgewandelt
zu $CO_2$ und $CH_4$

Annahme:

50% Kohlenstoff wird fermentiert
und
50% Kohlenstoff wird fossilisiert

150 kg C
fossilisiert

entspricht 540 kg
„captured and stored" $CO_2$
nach dem Kollabieren der
Salzkaverne

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20020111527 A
- EP 0320393 A1